# EUROPEAN PATENT SPECIFICATION

(11) **EP 1 948 099 B1**
(45) Date of publication and mention of the grant of the patent: **15.05.2019**
(21) Application number: 06803218.4
(22) Date of filing: 11.09.2006
(51) Int. Cl.: A61F 2/95

(54) **DOUBLE METAL STENT INTRODUCER**
DOPPEL-METALL-STENT-EINFÜHRGERÄT
INTRODUCTEUR METALLIQUE DOUBLE DE STENTS

(30) Priority: 16.09.2005 US 717689 P
(43) Date of publication of application: 30.07.2008
(73) Proprietor: Cook Medical Technologies LLC, Bloomington, IN 47404 (US)
(72) Inventor: KARPIEL, John, A., Winston-Salem, NC 27106 (US)
(74) Representative: Garratt, Peter Douglas
(86) International application number: PCT/US2006/035054
(87) International publication number: WO 2007/035287

(56) References cited:
- WO-A-01/03762
- WO-A-99/47071
- WO-A-99/51166
- WO-A2-02/30329
- WO-A2-99/56809

## Description

### RELATED APPLICATION

This application claims the benefit of priority from U.S. Provisional Application No. 60/717,689, filed September 16, 2005, and entitled "Double Metal Stent Introducer".

### TECHNICAL FIELD

The present invention relates to an apparatus for delivering an implantable prosthesis and, in particular, to an apparatus for simultaneously introducing multiple self-expanding stents to desired locations within a duct.

### BACKGROUND OF THE INVENTION

Self-expanding stents are used for a variety of applications. For example, self-expanding stents are used within the biliary tree to open strictures and maintain the patency of the duct. Current biliary and pancreatic self-expanding stent delivery systems generally include an introducer catheter with a single self-expanding stent loaded at the distal end thereof. A pusher catheter is used to deploy the stent from the distal end of the introducer catheter. In particular, the introducer catheter is retracted in a proximal direction relative to the pusher catheter to expose the stent from the end of the introducer catheter. As the stent is exposed from the end of the introducer catheter, it expands to engage the interior of the duct. The introducer and pusher catheters are then retracted and removed from the patient.

While current stent delivery systems are effective, they are limited to delivering a single self-expanding stent at one time. Thus, if a second stent is required to be deployed at or near the target site within the patient, the original stent delivery system must be retracted and removed from the patient before a second stent delivery system can be introduced and directed back to the target site within the patient. This can be a time consuming task because of the time required to first remove the original stent delivery system and then deliver the second stent delivery system to the target site. In addition, removal and replacement of the original stent delivery system may result in the loss of access to the target site. For example, the wire guide over which the stent delivery system is typically advanced and retracted may be inadvertently pulled from the target site during the exchange of stent delivery systems. If this occurs, then access to the target site will need to be re-established, thereby further increasing the duration and complexity of the medical procedure.

One example of a procedure requiring the deployment of two or more self-expanding stents is the treatment of a stricture in each leg of a bifurcated duct. As explained above, current self-expanding stent delivery systems can only deploy a single self-expanding stent. Thus, it is necessary to introduce a first stent delivery system into the first leg of the bifurcated duct and deploy the first stent therein. The first stent delivery system is then retracted and removed from the patient before a second stent delivery system can be introduced into the second leg of the bifurcated duct to deploy the second stent therein. Systems for deploying multiple stents or bifurcated stents are known e.g. from WO 99/47071 and WO 02/30329.

What is needed is a stent delivery system that can simultaneously deliver multiple self-expanding stents to a target site within a patient. What is also needed is a stent delivery system that can simultaneously deliver a self-expanding stent into each leg of a bifurcated duct. The present invention meets this need by providing an apparatus and method for deploying multiple self-expanding stents to one or more target sites within a patient, including each leg of a bifurcated duct.

### SUMMARY OF THE INVENTION

Accordingly, the stent introducer apparatus of the present invention comprises a primary stent introducer device movably disposed within a secondary stent introducer device. The primary stent introducer device includes a first self-expanding stent mounted on an elongate pusher member. The primary stent introducer device further includes an inner introducer catheter slidably disposed about the pusher member and the first stent. The inner introducer catheter, when disposed about the first stent, maintains the first stent in a compressed pre-deployment state. The pusher member is configured to hold the first stent stationary during movement of the inner introducer catheter relative to the pusher member and the first stent. The first stent is deployed by retracting the inner introducer catheter proximally relative to the pusher member to thereby expose the first stent from the distal end of the inner introducer catheter. Once exposed, the first stent self-expands to its deployed state.

The secondary stent introducer device includes a second self-expanding stent mounted within an outer introducer catheter. The outer introducer catheter, when disposed about the second stent, maintains the second stent in a compressed pre-deployment state. The outer introducer catheter is slidably disposed about the inner introducer catheter of the primary stent introducer device. The primary stent introducer device is configured to engage and hold the second stent stationary during movement of the outer introducer catheter relative to the primary stent introducer device and the second stent. The second stent is deployed by retracting the outer introducer catheter proximally relative to the primary stent introducer device to thereby expose the second stent from the distal end of the outer introducer catheter. Once exposed, the second stent self-expands to its deployed state.

In a first embodiment of the invention, the pusher member comprises a distal end cap configured to engage the proximal end of the second stent. In particular, the distal end cap comprises an outer flange that closely but slidably engages with the interior surface of the outer introducer catheter. A support element projects distally from the distal face of the flange and is spaced inwardly from the interior surface of the outer introducer catheter a distance sufficient to accommodate the second stent therebetween. The support element helps to align and maintain the proximal end of the second stent against the flange during deployment of the second stent. In an alternative embodiment of the invention, the outer periphery of distal end cap is disposed within the distal end of the inner introducer catheter. In this alternative embodiment, the proximal end of the second stent is engaged by the distal end of the inner introducer catheter during deployment of the second stent.

In the embodiment of the invention, the outer introducer catheter comprises a side port through the side wall thereof. The side port is located proximal to and near the proximal end of the second stent, and is sized to allow passage of the primary stent introducer device therethrough. In one aspect of the invention, the secondary stent introducer device is positioned within one leg of a bifurcated duct, while the primary stent introducer device is extended out through the side port and positioned within a second leg of the bifurcated duct.

In the first embodiment of the invention, the pusher member of the primary stent introducer device includes a lumen extending therethrough. The lumen is sized to accommodate a wire guide that may be used to introduce and position the stent introducer apparatus. For example, the wire guide may be used to position the secondary introducer device within one leg of a bifurcated duct. The wire guide may then be used to position the primary introducer device within another leg of the bifurcated duct. The lumen may also be used to introduce fluids to or remove fluids from the target site. For example, the lumen may be used to inject contrast media into the target site to facilitate fluoroscopic viewing of the area.

According to one aspect of the invention, the stent introducer apparatus is used to deploy a self-expanding stent into each of two legs of a bifurcated duct. The stent introducer apparatus is first introduced into the patient by passing it over a previously placed wire guide. This is accomplished by inserting the proximal end of the wire guide into the distal end of the stent introducer apparatus and through the lumen of the pusher member. The distal portion of the stent introducer apparatus, and more particularly the distal portion of the secondary stent introducer device, is advanced along the wire guide until it is positioned in one leg of a bifurcated duct. The distal end of the wire guide is then withdrawn into the distal end of the secondary stent introducer device to a location proximal of the side port in the outer introducer catheter. The wire guide is then advanced out through the side port and into the other leg of the bifurcated duct. Once the distal end of the wire guide has been positioned within the target site in the second duct, e.g., adjacent a stricture, the primary stent introducer device is extended along the wire guide and into the second duct. The first stent is then positioned and deployed at the target site by retracting the inner introducer catheter proximally relative to the pusher member to expose the stent from the distal end thereof.

Once the first stent has been deployed, the primary stent introducer device is retracted back through the side port and into the outer introducer catheter. The wire guide is likewise retracted back through the side port and into the outer introducer catheter. The primary stent introducer device is then advanced distally through the outer introducer catheter, past the side port, and into engagement with the second stent. If desired, the wire guide may then be re-advanced through and beyond the distal end of the outer introducer catheter to allow for re-positioning of the secondary stent introducer device within the first duct. The second stent is then positioned and deployed at the target site of the first duct by retracting the outer introducer catheter proximally relative to the primary stent introducer device, which acts as a pusher for the second stent, to expose the second stent from the distal end thereof. The entire stent introducer apparatus may then be retracted and removed from the patient.

According to another aspect of the invention, the stent introducer apparatus is used to deploy two self-expanding stents into a single duct. The stent introducer apparatus is introduced into the patient by passing it over a previously placed wire guide. This is accomplished by inserting the proximal end of the wire guide into the distal end of the stent introducer apparatus and through the lumen of the pusher member. The distal portion of the stent introducer apparatus, and more particularly the distal portion of the secondary stent introducer device, is advanced along the wire guide until it is positioned at a first target site in the duct. The primary stent introducer device is advanced distally through the outer introducer catheter, past the side port, and into engagement with the second stent. The second stent is then positioned and deployed at the first target site by retracting the outer introducer catheter proximally relative to the primary stent introducer device, which acts as a pusher for the second stent, to expose the second stent from the distal end thereof.

Once the second stent has been deployed, the primary stent introducer device is advance distally relative to the outer introducer catheter until the first stent is positioned beyond the distal end of the outer introducer catheter. The first stent is then positioned and deployed at the second target site within the duct by retracting the inner introducer catheter proximally relative to the pusher member to expose the stent from the distal end thereof. The entire stent introducer apparatus may then be retracted and removed from the patient.

These and other advantages, as well as the invention itself, will become apparent in the details of construction and operation as more fully described below. Moreover, it should be appreciated that several aspects of the invention can be used with other types of stent delivery catheters or medical devices.

### BRIEF DESCRIPTION OF THE DRAWINGS

FIG. 1 is a partially sectioned side view of one embodiment of the stent introducer apparatus of the present invention.
FIGS. 2-9 illustrate successive steps in one method of using the embodiment of the stent introducer apparatus of FIG. 1 to deploy a self-expanding stent into each of two legs of a bifurcated duct, wherein:

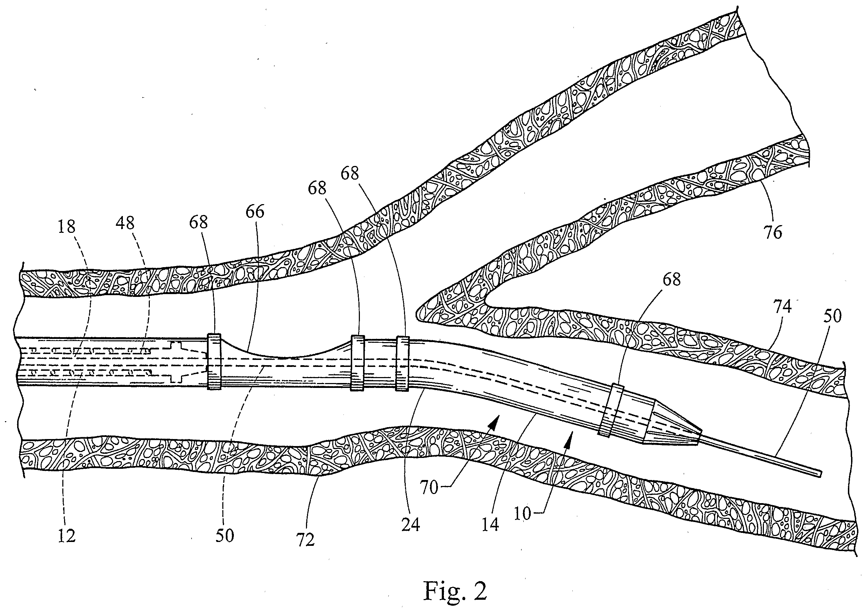

FIG. 2 illustrates the distal portion of the secondary stent introducer device positioned in a first leg of a bifurcated duct;

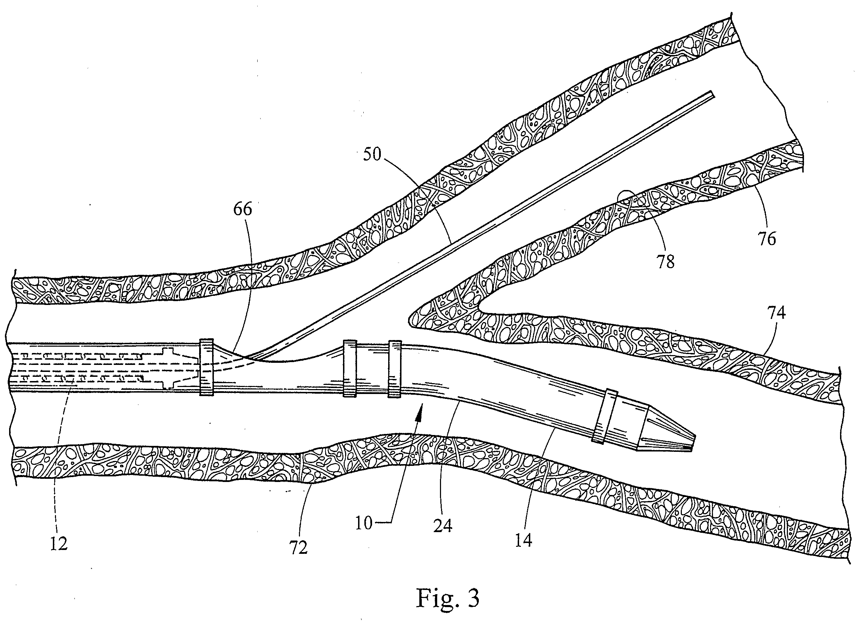

FIG. 3 illustrates a step following the step illustrated in FIG. 2 wherein the wire guide has been extended out through the side port in the outer introducer catheter and into a second leg of the bifurcated duct;

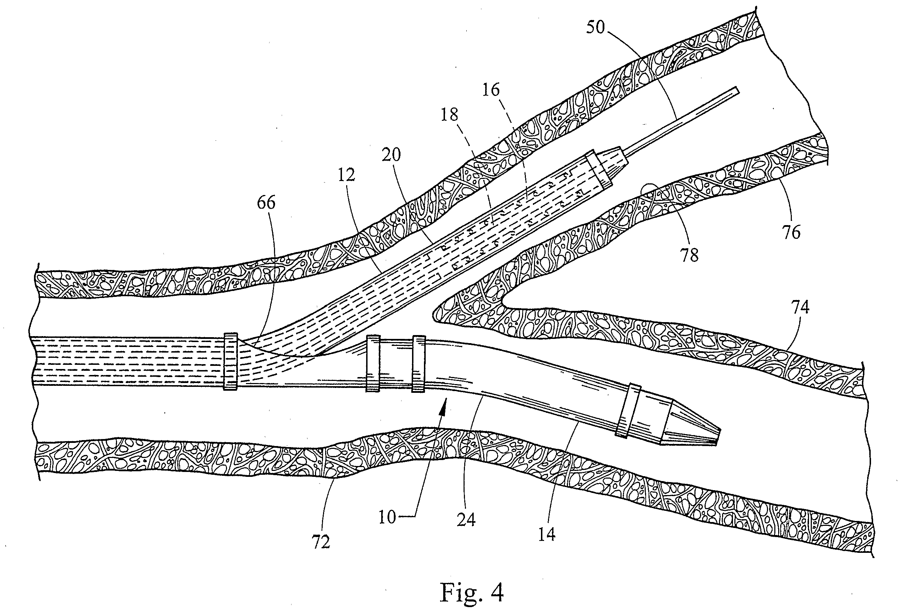

FIG. 4 illustrates a step following the step illustrated in FIG. 3 wherein the primary stent introducer device has been extended out through the side port in the outer introducer catheter and into the second leg of the bifurcated;

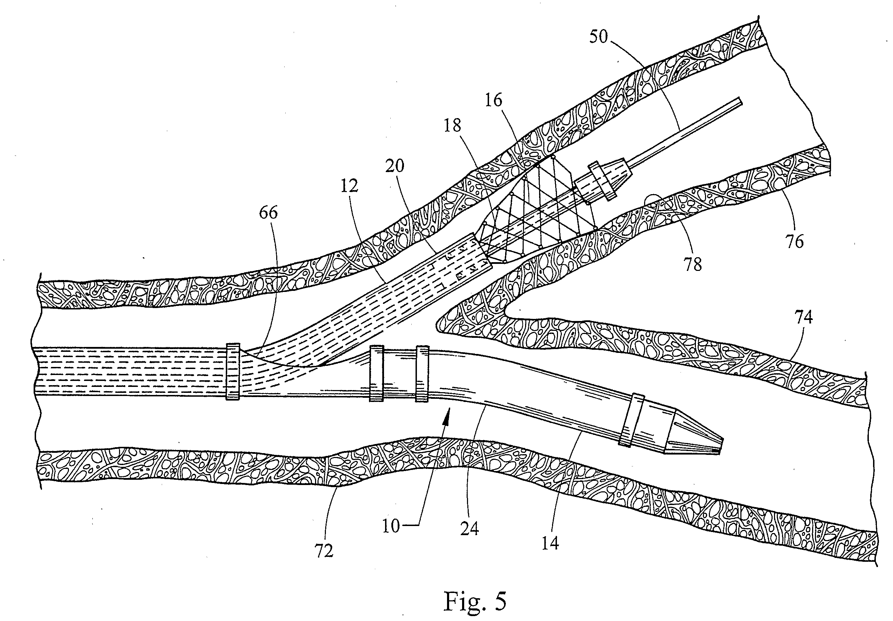

FIG. 5 illustrates a step following the step illustrated in FIG. 4 wherein the inner introducer catheter is being retracted to deploy the first stent in the second leg of the bifurcated duct;

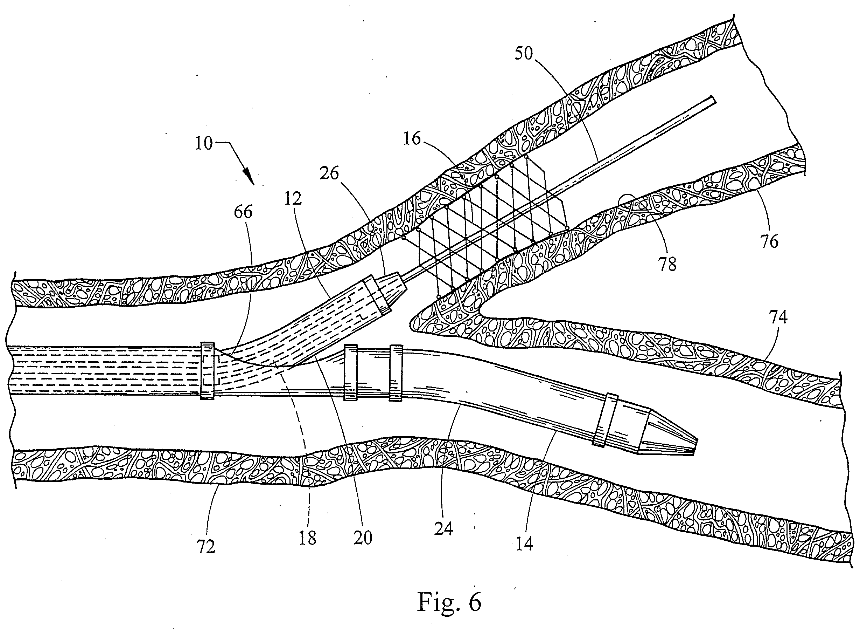

FIG. 6 illustrates a step following the step illustrated in FIG. 5 wherein the primary stent introducer device is being retracted back through the side port in the outer introducer catheter;

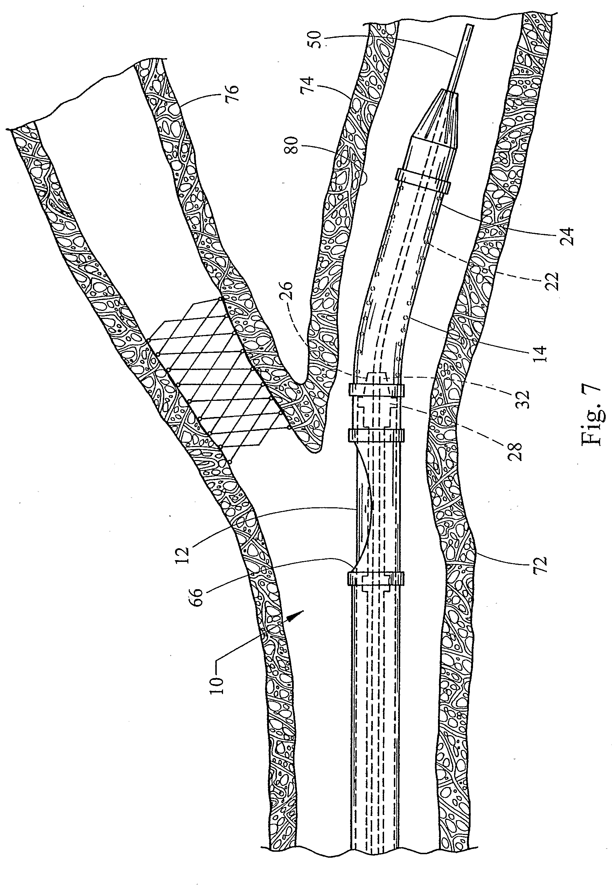

FIG. 7 illustrates a step following the step illustrated in FIG. 6 wherein the primary stent introducer device has been extended through the outer introducer catheter, past the side port and into engagement with the second stent;

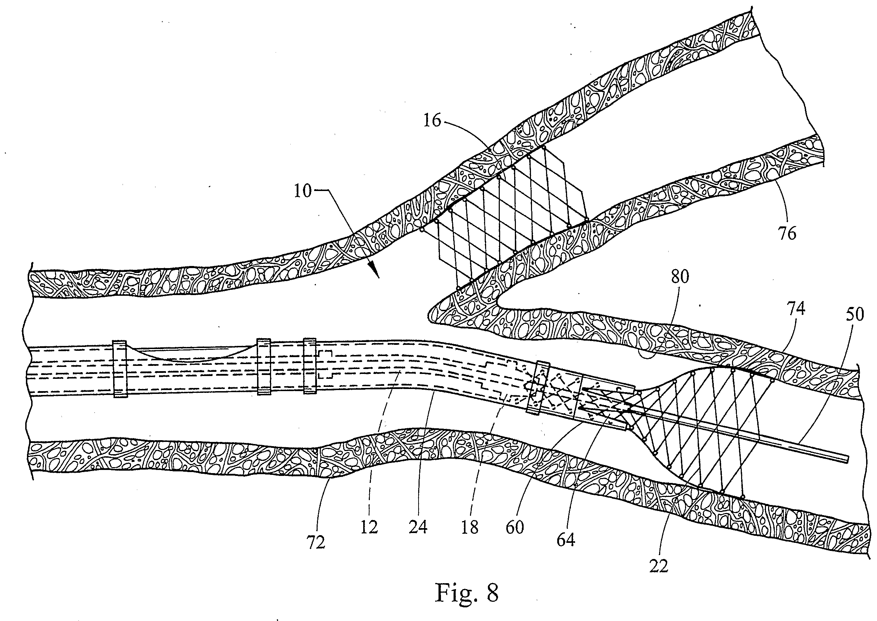

FIG. 8 illustrates a step following the step illustrated in FIG. 7 wherein the outer introducer catheter is being retracted to deploy the second stent in the first leg of the bifurcated duct; and

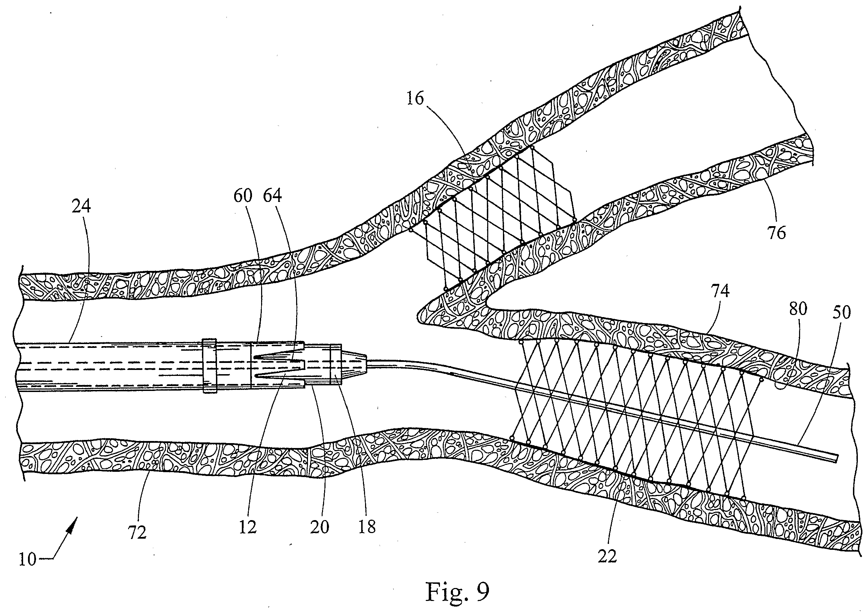

FIG. 9 illustrates a step following the step illustrated in FIG. 8 wherein the stent introducer apparatus is being retracted from the bifurcated duct.

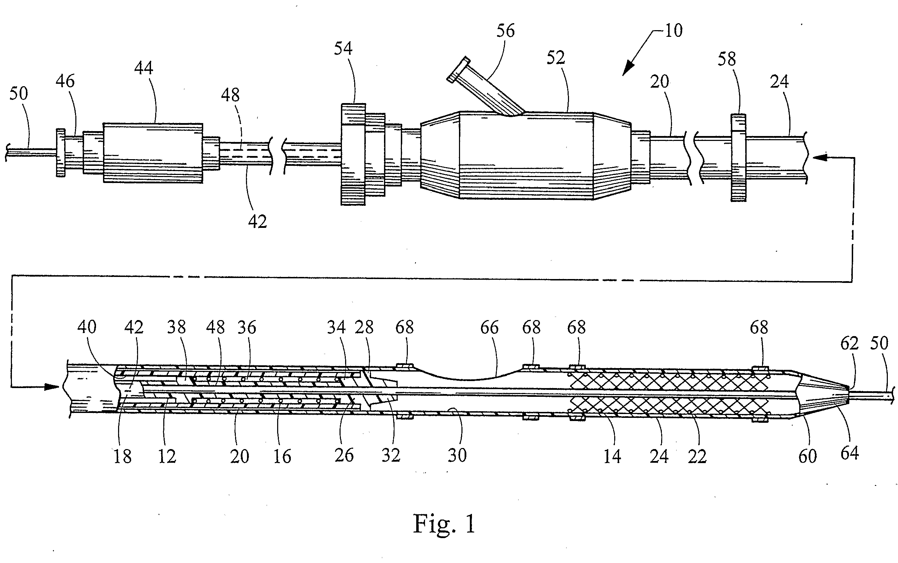

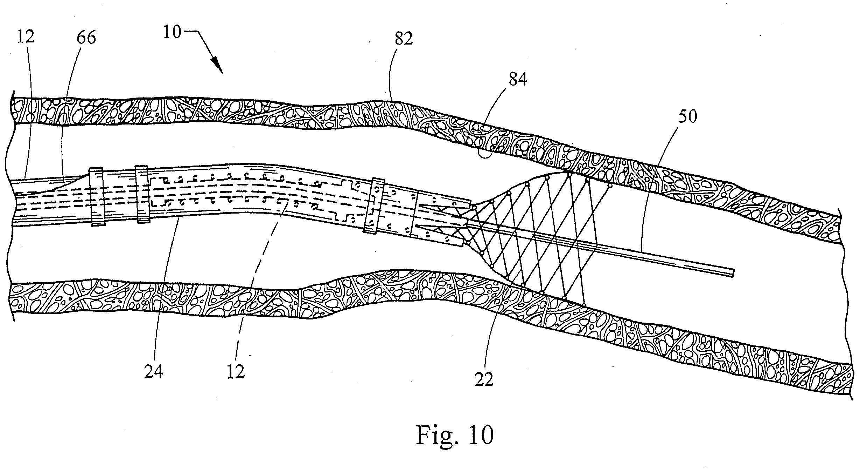

FIGS. 10-11 illustrate successive steps in another method of using the embodiment of the stent introducer apparatus of FIG. 1 to deploy two self-expanding stents into a single duct, wherein:
FIG. 10 illustrates the deployment of the second stent in the duct; and

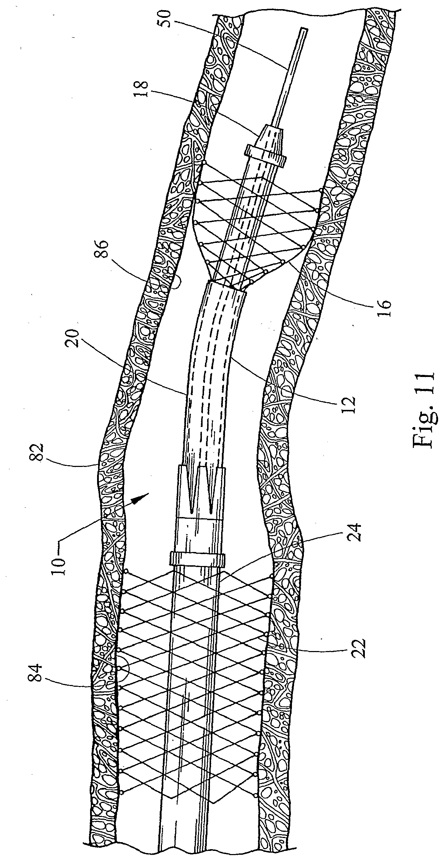

FIG. 11 illustrates the subsequent deployment of the first stent in the duct.

### DESCRIPTION OF THE INVENTION

Turning now to FIG. 1, one embodiment of the stent introducer apparatus 10 according to the present invention is shown. The stent introducer apparatus 10 comprises a primary stent introducer device 12 movably disposed within a secondary stent introduce device 14. The primary introducer device 12 includes a first self-expanding stent 16 mounted on an elongate pusher member 18. The primary stent introducer device 12 further includes an inner introducer catheter 20 slidably disposed about pusher member 18 and the first stent 16. The inner introducer catheter 20, when disposed about the first stent 16, maintains the first stent 16 in a compressed pre-deployment state. The pusher member 18 is configured to hold the first stent 16 stationary during movement of the inner introducer catheter 20 relative to the pusher member 18 and the first stent 16. The first stent 16 is deployed by retracting the inner introducer catheter 20 proximally relative to the pusher member 18 to thereby expose the first stent 16 from the distal end of the inner introducer catheter 20. Once exposed, the first stent 16 self-expands to its deployed state.

The secondary stent introducer device 14 includes a second self-expanding stent 22 mounted within an outer introducer catheter 24. The outer introducer catheter 24, when disposed about the second stent 22, maintains the second stent 22 in a compressed pre-deployment state. The outer introducer catheter 24 is slidably disposed about the inner introducer catheter 20 of the primary stent introducer device 12. The primary stent introducer device 12 is configured to engage and hold the second stent 22 stationary during movement of the outer introducer catheter 24 relative to the primary stent introducer device 12 and the second stent 22. The second stent 22 is deployed by retracting the outer introducer catheter 24 proximally relative to the primary stent introducer device 12 to thereby expose the second stent 12 from the distal end of the outer introducer catheter 24. Once exposed, the second stent 22 self-expands to its deployed state.

First and second stents 16, 22 each comprise a self-expanding prosthesis, which could include a self-expanding stent, such as the ZILVER™ Biliary Self-Expanding Stent, the SPIRAL Z™ self-expanding stent, and the ZA-STENT™ self-expanding stent, each of which are sold by Wilson-Cook Medical Inc., d/b/a as Cook™ Endoscopy. However, first and second stents 16, 22 could comprise any nitinol, stainless steel, or other self-expanding stent; artificial valve (e.g., venous, heart, pulmonary, etc.) prosthesis, vessel occluder, filter, embolic protection device, shunt, stent graft, etc., that is capable of self-expanding from a compressed pre-deployment (or delivery) state to an expanded deployed state. As such, the term "stent" as used herein refers generally to, but is not limited to, any of the self-expanding devices described above. It is also contemplated that non-expanding stents could also be utilized in the introducer apparatus of the present invention.

In the embodiment illustrated in FIG. 1, the pusher member 18 comprises a distal end cap 26 configured to engage the proximal end of the second stent 22. In particular, the distal end cap 26 comprises an outer flange 28 that closely but slidably engages with the interior surface 30 of the outer introducer catheter 24. A support element 32 projects distally from the distal face of the flange 28 and is spaced inwardly from the interior surface 30 of the outer introducer catheter 24 a distance sufficient to accommodate the second stent 22 therebetween. In other words, a gap is provided about the exterior surface of the support element 32 that is equal to or larger than the wall thickness of the second stent 22 when in the compressed pre-deployment state. As will be explained in greater detail below, the support element 32 helps to align and maintain the proximal end of the second stent 16 against the flange 28 during deployment of the second stent 22. The support element 32 is preferably rounded or tapered towards the distal end thereof so as to prevent the distal end from contacting and possibly dislodging the second stent 22 as the primary stent introducer device 12 is brought into engagement with the second stent 22. The rounded or tapered end also facilitates an atraumatic introduction of the primary stent introducer device 12 when the primary stent introducer device 12 is extended outwardly from the secondary stent introducer device 14 and advanced through the lumen or duct of a patient.

In the embodiment illustrated, the support element 32 has a length that will only partially extend into the second stent 22 when the primary stent introducer device 12 is brought into engagement with the second stent 22. However, the support element 32 may have a longer length that is sufficient to extend through the entire second stent 22. This longer length would permit the support element 32 to support the interior of the second stent 22 and prevent the second stent from kinking, buckling or otherwise deforming during its deployment.

The distal end cap 26 further comprises a collar 34 extending proximally from the proximal face of the flange 28. The collar 34 is spaced inwardly from the interior surface 30 of the outer introducer catheter 24 a distance sufficient to accommodate the inner introducer catheter 20 therebetween. In other words, a gap is provided about the exterior surface of the collar 32 that is equal to or larger than the wall thickness of the inner introducer catheter 20. The collar 34 allows the inner introducer catheter 20 to overlap with the distal end cap 26 a distance sufficient to prevent the first stent 16 from being exposed should the inner introducer catheter 20 inadvertently move proximally relative to the pusher member 18 during introduction, advancement and manipulation of the stent introducer apparatus 10. The overlap also provides a seal between the distal end cap 26 and the inner introducer catheter 20 to prevent the first stent 16 from being exposed to bodily fluids or contaminants.

In an alternative embodiment of the invention not illustrated, the outer periphery of distal end cap 26 is completely disposed within the distal end of the inner introducer catheter 20. In this alternative embodiment, and as will be explained in greater detail below, the proximal end of the second stent 22 is engaged by the distal end of the inner introducer catheter 20 during deployment of the second stent 22.

The pusher member 18 further comprises a stent carrying region 36. The stent carrying region 36 is disposed proximally of the distal end cap 26 and comprises a recess having a sufficient depth and length to accommodate the first stent 16 in a compressed pre-deployment state within the interior of the inner introducer catheter 20. In other words, the stent carrying region 36 of the pusher member 18, together with the interior surface 40 of the inner introducer catheter 20, forms a tubular volume within which the first stent 16 is disposed.

The pusher member 18 further comprises a stent pusher 38 disposed adjacent to the proximal end of the stent carrying region 36. The stent pusher 38 is sized to closely but slidably engage with the interior surface 40 of the inner introducer catheter 20. As will be explained in greater detail below, the stent pusher 38 engages the proximal end of the first stent 16 and holds the first stent 16 stationary (relative to the pusher member 18) as the inner introducer catheter 20 is retracted (relative to the pusher member 18) to deploy the first stent 16.

Portions of the distal portion of the pusher member 18 may include barium sulfate or some other agent or marker to provide radiopacity to the primary stent introducer device 12. For example, the distal end cap 26 and the stent pusher 38 can each be made radiopaque so as to enable the user, with the aid of a fluoroscope, to determine the position of the first stent 16 prior to deployment.

The pusher member 18 further comprises an elongate rod 42 extending between the stent pusher 38 and a handle 44 attached to the proximal end of the pusher member 18. The rod 42 is typically, but not necessarily, smaller in cross-section than the interior cross-section of the inner introducer catheter 20 to reduce frictional forces that may be generated by the axial or rotational movement of the pusher member 18 relative to the inner introducer catheter 20. The rod 42 comprises a sufficient rigidity to transmit axial and/or torsional forces between the stent pusher 38 and the handle 44 without excessive bending, buckling or twisting. On the other hand, the rod 42 must be sufficiently flexible to permit passage of the stent introducer apparatus 10 through the patient's bodily lumens. Suitable materials include, but are not limited to, PEEK, polyvinyl chloride (PVC), polyimide, polyimide reinforced with a stainless steel braid, polyurethane, nylon, metal tubing such as nitinol or stainless steel, and the like. The rod 42 may also be formed as a coil or a solid-core wire guide. It should be understood that the rod 42 may be formed integrally with the handle 44 and/or the distal-most portions of the pusher member 18 such as the distal end cap 26, the stent carrying region 36, and the stent pusher 38. In one embodiment, a substantial portion of the rod 42 is formed from nylon tubing while the distal portion, and especially the stent-carrying region, is formed from polyimide.

In the embodiment illustrated, the pusher member 18 comprises a lumen 48 extending through the length of the pusher member 18. The lumen 48 is sized to accommodate a wire guide 50 that may be used to introduce, advance and position the stent introducer apparatus 10 within the patient. For example, and as will be explained in greater detail below, the wire guide 50 may be used to position the secondary stent introducer device 14 within a first leg of a bifurcated duct. The wire guide 50 may then be used to position the primary stent introducer device 12 within the second leg of the bifurcated duct. The lumen 48 may also be used to introduce or remove fluids to or from the target site within the patient. For example, the lumen 48 may be used to inject contrast media into the target site to facilitate fluoroscopic viewing of the area. The handle 44 of the pusher member 18 comprises a fitting or seal 46 that cooperates with a lumen 48. The seal 46 prevents fluids that may enter the distal end of the lumen 48 from passing out through the handle 44 at the proximal end of the lumen 48. The seal 46 comprises a material, or has an adjustable opening, that will accommodate and seal about a wire guide 50 passing therethrough.

The inner introducer catheter 20 generally comprises an elongated tubular structure or sheath made from any suitable material that is sufficiently flexible to permit passage of the stent introducer apparatus 10 through the patient's bodily lumens. The material should also be sufficiently inelastic to resist undue elongation caused by the application of tensile forces at the proximal end of the inner introducer catheter 20 during deployment of the first stent 16. The material may also be made from a clear material such as a substantially clear polymer. Providing a transparency to the inner introducer catheter 20 enables the user to inspect the first stent 16 prior to deployment. Suitable materials include, but are not limited to, polytetrafluoroethylene (PTFE) and polyeretherketone (PEEK).

The inner introducer catheter 20 further comprises a handle 52 attached to the proximal end of the inner introducer catheter 20. The handle 52 attached to the inner introducer catheter 20, in cooperation with the handle 44 attached to the pusher member 18, facilitates movement of the inner introducer catheter 20 relative to the pusher member 18. In particular, and as will be discussed in greater detail below, the user grips and moves handles 44 and 52 towards each other to retract the inner introducer catheter 20 proximally relative to the pusher member 18 to deploy first stent 16. Prior to deployment, handles 44 and 52 are spaced a sufficient distance apart to permit the inner introducer catheter 20 to be moved proximally relative to the pusher member 18 so as to allow the distal end of the inner introducer catheter 20 to be moved proximally of the first stent 16.

The handle 52 of the inner introducer catheter comprises a seal 54 and a fluid injection port 56. The fluid injection port 56 provides a mechanism for flushing the cavity between the inner introducer catheter 20 and the pusher member 18. In particular, a syringe or similar device (not shown) is attached to the fluid injection port 56 and saline is injected therethrough. The saline flushes any air that is trapped within the inner introducer catheter 20 out though the distal end thereof. The seal 54 prevents the saline from escaping out from the proximal end of the handle 52 (about the rod 42) during the flushing procedure.

The outer introducer catheter 24 generally comprises an elongated tubular structure or sheath made from any suitable material that is sufficiently flexible to permit passage of the stent introducer apparatus 10 through the patient's bodily lumens. The material should also be sufficiently inelastic to resist undue elongation caused by the application of tensile forces at the proximal end of the outer introducer catheter 24 during deployment of the second stent 22. The material may also be made from a clear material such as a substantially clear polymer. Providing a transparency to the outer introducer catheter 20 enables the user to inspect the second stent 22, as well as the position of the primary stent introducer device 12, prior to deployment of the second stent 22. Suitable materials include, but are not limited to, polytetrafluoroethylene (PTFE) and polyeretherketone (PEEK).

The outer introducer catheter 24 further comprises a handle 58 attached to the proximal end of the outer introducer catheter 24. The handle 58 attached to the outer introducer catheter 24, in cooperation with the handle 44 attached to the pusher member 18, facilitates movement of the outer introducer catheter 24 relative to the pusher member 18. In particular, and as will be discussed in greater detail below, the user grips and moves handles 44 and 58 towards each other to retract the outer introducer catheter 24 proximally relative to the pusher member 18 to deploy second stent 22. Prior to deployment, handles 44 and 58 are spaced a sufficient distance apart to permit the outer introducer catheter 24 to be moved proximally relative to the pusher member 18 so as to allow the distal end of the outer introducer catheter 24 to be moved proximally of the second stent 22. In addition, it should be understood that the spacing of handles 44 and 58 must also be sufficient to accommodate the handle 52 of the inner introducer catheter 20, which is disposed therebetween.

The distal end 60 of the outer introducer catheter 24 is tapered or rounded to facilitate an atraumatic introduction of the secondary stent introducer device 14 through the patient's bodily lumens. An opening 62 is provided in the distal end 60 to allow the passage of wire guide 50 therethrough. The distal end 60 further comprises weakened areas or slits 64 that allow the distal end to spread apart during deployment of the second stent 22. In particular, slits 64 allow the tapered distal end 60 to spread apart and assume a tubular cross-section substantially equal to the portion of the outer introducer catheter 24 located proximally thereof.

In the embodiment illustrated, the outer introducer catheter 24 comprises a side port 66 through the side wall thereof. The side port 66 is located proximal to the second stent 22, and is preferably located near the proximal end of the second stent 22. However, side port 66 could be spaced away from the proximal end of the second stent 22 by any practical distance. Alternatively, the side port 66 could located distal to the second stent 22, e.g., in or near the tapered end 60. Side port 66 is sized to allow passage of the primary stent introducer device 12 therethrough. In particular, and as will be explained in greater detail below, the primary stent introducer device 12 may be extended out through the side port 66 and positioned within one leg of a bifurcated duct while the secondary stent introducer device 14 is positioned within the other leg of the bifurcated duct. Thus, the side port 66 must be large enough to permit the primary stent introducer device 12 to freely pass therethrough without kinking or excessively bending the primary stent introducer device 12 or the outer introducer catheter 24. In the embodiment illustrated, the side port 66 comprises an elongated opening or slot. The portion of the outer introducer catheter 24 adjacent or surrounding the side port 66 may be reinforced to prevent kinking or excessive bending.

The outer introducer catheter 24 includes radiopaque markers 68 space along the surface thereof. In the embodiment illustrated, one pair of markers 68 are located near the ends of the second stent 22, and another set of markers 68 are located nears the ends of the side port 66. These markers 68 assist the user, with the aid of fluoroscopy, to locate and position the outer introducer catheter 24 during various stages of the stent deployment procedure.

As best seen in FIG. 2, the distal portion of the outer introducer catheter 24 (e.g., the portion distal of the side port 66) may be bendable or pre-bent. As will be explained in greater detail below, a curved distal section 70 may facilitate the proper orientation and positioning of the secondary stent introducer device 14 in one leg of a bifurcated duct, while at the same time orienting the side port 66 to align with the other leg of the bifurcated duct. Thus, the curved distal section 70 is preferably oriented so as to bias the distal end 60 in a direction that is opposite of the side on which the side port 66 is disposed.

According to one aspect of the invention, the stent introducer apparatus 10 is used to deploy a self-expanding stent into each of two legs of a bifurcated duct. An exemplary method for performing this procedure is illustrated in FIGS. 2-9, which illustrate successive steps in this exemplary method of using the embodiment of the stent introducer apparatus of FIG. 1 to deploy a self-expanding stent into each of two legs of a bifurcated duct.

As illustrated in FIG. 2, the stent introducer apparatus 10 is first introduced into the patient by passing it over a previously placed wire guide 50. In particular, the wire guide 50 is first inserted into the patient and advanced through the patient's bodily lumens until it reaches the target bifurcated duct 72. The use of an endoscope (not shown) or other introducer device may be employed to assist in the introduction, advancement, and placement of the wire guide 50. The distal end of the wire guide 50 is then positioned within the first leg 74 of the bifurcated duct 72. The stent introducer apparatus 10 is then advanced over the wire guide 50 and then likewise positioned in the first leg 74 of the bifurcated duct 72. This is accomplished by first inserting the proximal end of the wire guide 50 into the distal end 60 of the outer introducer catheter 24 and through the lumen 48 of the pusher member 18. The distal portion of the stent introducer apparatus 10, and more particularly the distal portion of the secondary stent introducer device 14, is then advanced along the wire guide 50 until it is position in the first leg 74 of the bifurcated duct 72. Preferably, side port 66 is position and oriented so as to align with the second leg 76 of the bifurcated duct 72. Radiopaque markers 68 may be utilized, with the aid of fluoroscopy, to assist in the proper positioning and orientation of the stent introducer apparatus 10.

The distal end of the wire guide 50 is then withdrawn into the distal end of the secondary stent introducer device 14 to a location proximal of the side port 66 in the outer introducer catheter 24. As illustrated in FIG. 3, the wire guide 50 is then advanced out through the side port 66 and into the second leg 76 of the bifurcated duct 72. A curved or pre-bent tip or distal end of the wire guide 50 may facilitate its manipulation through side port 66. The wire guide 50 is then advanced so as to extend past the target site within the second leg 76 of the bifurcated duct 72, e.g., past stricture 78.

As illustrated in FIG. 4, the primary stent introducer device 12 is then extended along the wire guide 50 and into the second leg 76 of the bifurcated duct 72. In particular, the primary stent introducer device 12 is advanced through the side port 66 of the outer introducer catheter 24. The distal portion of the primary stent introducer device 12 is then positioned so that the first stent 16 is located at the target site, e.g., along stricture 78. Radiopaque markers or portions of the primary stent introducer device 12 may be utilized to facilitate its positioning.

As illustrated in FIG. 5, the first stent 16 is then deployed at the target site by retracting the inner introducer catheter 20 proximally relative to the pusher member 18 to expose the first stent 16 from the distal end thereof. As explained above, inner introducer catheter 20 is retracted proximally relative to the pusher member 18 by moving handle 52 towards handle 44 (see FIG. 1).

As illustrated in FIG. 6, once the first stent 16 has been deployed, the primary stent introducer device 12 is retracted back through the side port 66 and into the outer introducer catheter 24. Although not necessary to the procedure, the inner introducer catheter 20 may be advanced distally relative to the pusher member 18 (or the pusher member 18 retracted proximally relative to the inner introducer catheter 20) so as to re-engage the distal end of the inner introducer catheter 20 with the distal end cap 26 of the pusher member 18. The wire guide 50 is likewise retracted back through the side port 66 and into the outer introducer catheter 24.

As illustrated in FIG. 7, the primary stent introducer device 12 is then advanced distally through the outer introducer catheter 24, past the side port 66 and into engagement with the second stent 22. In particular, the support element 32 of the distal end cap 26 is advanced into the interior volume of the second stent 22 until the flange 28 engages the proximal end of the second stent 22. If desired, the wire guide 50 may then be re-advanced through and beyond the distal end of the outer introducer catheter 24 to allow for re-positioning of the secondary stent introducer device 14 within the first leg 74 of the bifurcated duct 72. In other words, it may be necessary to advance the secondary stent introducer device 14 further into the first leg 74 so as to position the second stent 22 within the target site, e.g., along stricture 80.

As illustrated in FIG. 8, the second stent 22 is then positioned and deployed at the target site of the first leg 74 of the bifurcate duct 72 by retracting the outer introducer catheter 24 proximally relative to the primary stent introducer device 12, which acts as a pusher for the second stent 22, so as to expose the second stent 22 from the distal end thereof. More specifically, the outer introducer catheter 24 is retracted proximally relative to the pusher member 18 by moving handle 58 proximally towards handle 44. As explained above, the slits 64 in the distal end 60 of the outer introducer catheter 24 allow the distal end 60 to expand to move proximally past second stent 22 during deployment.

As illustrated in FIG. 9, the stent introducer apparatus 10 is then retracted from the bifurcated duct 72 and removed from the patient.

According to another aspect of the invention, the stent introducer apparatus 10 is used to deploy two self-expanding stents into a single duct. An exemplary method for performing this procedure is illustrated in FIGS. 10-11, which illustrate successive steps in this exemplary method of using the embodiment of the stent introducer apparatus of FIG. 1 to deploy two self-expanding stents into a single duct. Many of the steps in the exemplary method are similar or identical to steps in the exemplary method discussed above in connection with FIGS. 2-9 and therefore will not be repeated in the following description.

As illustrated in FIG. 10, the stent introducer apparatus 10 is introduced into the patient by passing it over a previously placed wire guide 50. In particular, the distal portion of the stent introducer apparatus 10, and more particularly the distal portion of the secondary stent introducer device 24, is advanced along the wire guide 50 until the second stent 22 is positioned at a first target site 84 in the duct 82. The primary stent introducer device 12 is then advanced distally through the outer introducer catheter 24, past the side port 66 and into engagement with the second stent 22. The second stent 22 is then positioned and deployed at the first target site 84 by retracting the outer introducer catheter 24 proximally relative to the primary stent introducer device 10, and more particularly the pusher member 18, which acts as a pusher for the second stent 22, so as to expose the second stent 22 from the distal end thereof.

As illustrated in FIG. 11, once the second stent 22 has been deployed, the primary stent introducer device 12 is advance distally relative to the outer introducer catheter 24 until the first stent 16 is positioned beyond the distal end of the outer introducer catheter 24. The first stent 16 is then positioned and deployed at the second target site 86 within the duct 82 by retracting the inner introducer catheter 20 proximally relative to the pusher member 18 to expose the first stent 16 from the distal end thereof. The entire stent introducer apparatus 10 may then be retracted and removed from the patient.

While there have been described what are presently believed to be the preferred embodiments of the invention, those skilled in the art will realize that changes and modifications may be made thereto without departing from the scope of the invention as defined in the claims.

## Claims

1. A stent introducer apparatus comprising:
a primary stent introducer (12) including an elongate pusher member (18), an elongate inner introducer catheter (20) slidably disposed about the pusher member, and a first prosthesis (16) removably mounted within the inner introducer catheter, the first prosthesis being expandable from a compressed pre-deployment configuration to an expanded deployed configuration, the first prosthesis being confined in the compressed pre- deployment configuration when disposed within the inner introducer catheter, the first prosthesis being permitted to expand to the expanded deployed configuration when not disposed within the inner introducer catheter; and
a secondary stent introducer (14) including an elongate outer introducer catheter (24) and a second prosthesis (22) removably mounted within the outer introducer catheter, the second prosthesis being disposed distally of the primary stent introducer, the outer introducer catheter being slidably disposed about the primary stent introducer, the second prosthesis being expandable from a compressed pre-deployment configuration to an expanded deployed configuration, the second prosthesis being confined in the compressed pre-deployment configuration when disposed within the outer introducer catheter, the second prosthesis being permitted to expand to the expanded deployed configuration when not disposed within the outer introducer catheter;
wherein the first prosthesis is deployed by retracting the inner introducer catheter proximally relative to the pusher member so as to expose the first prosthesis from a distal end of the inner introducer catheter, and
wherein the second prosthesis is deployed by retracting the outer introducer catheter proximally relative to the primary stent introducer so as to expose the second prosthesis from a distal end of the outer introducer catheter;
**characterised in that** the outer introducer catheter comprises a port (66) through a side wall of the outer introducer catheter, the side port proximal to the second prosthesis and configured to allow passage of the primary stent introducer therethrough.

2. The stent introducer apparatus according to claim 1, wherein the first prosthesis is removably mounted about the pusher member.

3. The stent introducer apparatus according to claim 2, wherein the pusher member comprises a stent carrying region and a stent pusher, the stent pusher being disposed proximally of the stent carrying region and configured to abut a proximal end of the first prosthesis, further wherein the first prosthesis is removably mounted about the stent carrying region when disposed within the inner introducer catheter.

4. The stent introducer apparatus according to claim 1, wherein the second prosthesis is deployed by retracting the outer introducer catheter proximally relative to the pusher member so as to engage the second prosthesis with the pusher member and expose the second prosthesis from a distal end of the outer introducer catheter.

5. The stent introducer apparatus according to claim 4, wherein the pusher member comprises a distal end cap including a flange and a support element, the flange configured to abut against a proximal end of the second prosthesis, the support element configured to extend into an interior volume of the second prosthesis.

6. The stent introducer apparatus according to claim 1, wherein the second prosthesis is deployed by retracting the outer introducer catheter proximally relative to the inner introducer catheter so as to engage the second prosthesis with the inner introducer catheter and expose the second prosthesis from a distal end of the outer introducer catheter.

7. The stent introducer apparatus according to claim 1, wherein the pusher member comprises a lumen extending through at least a portion thereof.

8. The stent introducer apparatus according to claim 7, further comprising a wire guide, the wire guide extending through at least a portion of the lumen.

9. The stent introducer apparatus according to claim 1, wherein the outer introducer catheter comprises a distal end, the distal end having a rounded or tapered profile.

10. The stent introducer apparatus according to claim 9, wherein the distal end is expandable to permit passage of the primary stent introducer therethrough.

11. The stent introducer apparatus according to claim 10, wherein the distal end comprises one or more slits extending at least partially through a surface thereof, the one or more slits be configured to permit the distal end to expand to permit passage of the primary stent introducer therethrough.

## Patentansprüche

1. Stent-Einführvorrichtung, umfassend:
ein primäres Stent-Einführgerät (12), das ein längliches Schiebeelement (18), einen länglichen inneren Einführkatheter (20), der verschiebbar um das Schiebeelement angeordnet ist, und eine erste Prothese (16) umfasst, die innerhalb des inneren Einführkatheters entfernbar angebracht ist, wobei die erste Prothese aus einer komprimierten Vorentfaltungsauslegung in eine aufgeweitete Nachentfaltungsauslegung aufgeweitet werden kann, wobei die erste Prothese in der komprimierten Vorentfaltungsauslegung eingeschlossen ist, wenn sie in dem inneren Einführkatheter angeordnet ist, wobei sich die erste Prothese in die aufgeweitete entfaltete Konfiguration aufweiten darf, wenn sie sich nicht innerhalb des inneren Einführkatheters befindet; und
ein sekundäres Stent-Einführgerät (14), das einen länglichen äußeren Einführkatheter (24) und eine zweite Prothese (22) umfasst, die innerhalb des äußeren Einführkatheters entfernbar angebracht ist, wobei die zweite Prothese distal zu dem primären Stent-Einführgerät angeordnet ist, wobei der äußere Einführkatheter verschiebbar um das primäre Stent-Einführgerät angeordnet ist, wobei die zweite Prothese aus einer komprimierten Vorentfaltungsauslegung in eine aufgeweitete Nachentfaltungsauslegung aufgeweitet werden kann, wobei die zweite Prothese in der komprimierten Vorentfaltungsauslegung eingeschlossen ist, wenn sie in dem äußeren Einführkatheter angeordnet ist, wobei sich die zweite Prothese in die aufgeweitete entfaltete Konfiguration aufweiten darf, wenn sie sich nicht innerhalb des äußeren Einführkatheters befindet;
wobei die erste Prothese durch proximales Zurückziehen des inneren Einführkatheters bezogen auf das Schiebeelement entfaltet wird, um die erste Prothese von einem distalen Ende des inneren Einführkatheters freizulegen, und
wobei die zweite Prothese durch proximales Zurückziehen des äußeren Einführkatheters bezogen auf das primäre Stent-Einführgerät entfaltet wird, um die zweite Prothese von einem distalen Ende des äußeren Einführkatheters freizulegen;
**dadurch gekennzeichnet, dass** der äußere Einführkatheter eine Öffnung (66) durch eine Seitenwand des äußeren Einführkatheters umfasst, wobei die Seitenöffnung proximal zu der zweiten Prothese und dazu ausgelegt ist, den Durchgang des primären Stent-Einführgeräts dort hindurch zu ermöglichen.

2. Stent-Einführvorrichtung nach Anspruch 1, wobei die erste Prothese um das Schiebeelement entfernbar angebracht ist.

3. Stent-Einführvorrichtung nach Anspruch 2, wobei das Schiebeelement einen den Stent tragenden Bereich und einen Stent-Schieber umfasst, wobei der Stent-Schieber proximal zu dem den Stent tragenden Bereich angeordnet und dazu ausgelegt ist, an einem proximalen Ende der ersten Prothese anzuliegen, wobei ferner die erste Prothese um den den Stent tragenden Bereich entfernbar angebracht ist, wenn sie innerhalb des inneren Einführkatheters angeordnet ist.

4. Stent-Einführvorrichtung nach Anspruch 1, wobei die zweite Prothese durch proximales Zurückziehen des äußeren Einführkatheters bezogen auf das Schiebeelement entfaltet wird, um die zweite Prothese mit dem Schiebeelement in Eingriff zu bringen und die zweite Prothese von einem distalen Ende des äußeren Einführkatheters freizulegen.

5. Stent-Einführvorrichtung nach Anspruch 4, wobei das Schiebeelement einen Distalendaufsatz umfasst, der einen Flansch und ein Tragelement umfasst, wobei der Flansch dazu ausgelegt ist, an einem proximalen Ende der zweiten Prothese anzuliegen, wobei das Tragelement dazu ausgelegt ist, sich in ein Innenvolumen der zweiten Prothese zu erstrecken.

6. Stent-Einführvorrichtung nach Anspruch 1, wobei die zweite Prothese durch proximales Zurückziehen des äußeren Einführkatheters bezogen auf den inneren Einführkatheter entfaltet wird, um die zweite Prothese mit dem inneren Einführkatheter in Eingriff zu bringen und die zweite Prothese von einem distalen Ende des äußeren Einführkatheters freizulegen.

7. Stent-Einführvorrichtung nach Anspruch 1, wobei das Schiebeelement ein Lumen umfasst, das sich durch mindestens einen Abschnitt davon erstreckt.

8. Stent-Einführvorrichtung nach Anspruch 7, ferner umfassend eine Drahtführung, wobei sich die Drahtführung durch mindestens einen Abschnitt des Lumens erstreckt.

9. Stent-Einführvorrichtung nach Anspruch 1, wobei der äußere Einführkatheter ein distales Ende umfasst, wobei das distale Ende ein abgerundetes oder konisches Profil aufweist.

10. Stent-Einführvorrichtung nach Anspruch 9, wobei das distale Ende aufweitbar ist, um den Durchgang des primären Stent-Einführgeräts dort hindurch zu erlauben.

11. Stent-Einführvorrichtung nach Anspruch 10, wobei das distale Ende einen oder mehrere Schlitze umfasst, die sich mindestens teilweise durch eine Oberfläche davon erstrecken, wobei der eine oder die mehreren Schlitze dazu ausgelegt sind, zu erlauben, dass sich das distale Ende aufweitet, um den Durchgang des primären Stent-Einführgeräts dort hindurch zu erlauben.

## Revendications

1. Appareil introducteur de stent comprenant :
un introducteur principal (12) de stent comprenant un élément poussoir allongé (18), un cathéter (20) d'introducteur interne allongé disposé coulissant autour de l'élément poussoir, et une première prothèse (16) montée amovible dans le cathéter d'introducteur interne, la première prothèse étant déployable depuis une configuration comprimée avant déploiement vers une configuration déployée détendue, la première prothèse étant confinée dans la configuration comprimée avant déploiement lorsqu'elle est disposée dans le cathéter d'introducteur interne, la première prothèse pouvant se détendre vers la configuration déployée détendue lorsqu'elle n'est pas disposée dans le cathéter d'introducteur interne ; et
un introducteur secondaire (14) de stent comprenant un cathéter (24) d'introducteur externe allongé et une seconde prothèse (22) montée amovible dans le cathéter d'introducteur externe, la seconde prothèse étant disposée distalement à l'introducteur principal de stent, le cathéter d'introducteur externe étant disposé coulissant autour de l'introducteur principal de stent, la seconde prothèse étant déployable depuis une configuration comprimée avant déploiement vers une configuration déployée détendue, la seconde prothèse étant confinée dans la configuration comprimée avant déploiement lorsqu'elle est disposée dans le cathéter d'introducteur externe, la seconde prothèse pouvant se détendre vers la configuration déployée détendue lorsqu'elle n'est pas disposée dans le cathéter d'introducteur externe ;
dans lequel la première prothèse est déployée par rétraction du cathéter d'introducteur interne proximalement par rapport à l'élément poussoir de façon à exposer la première prothèse depuis une extrémité distale du cathéter d'introducteur interne, et
dans lequel la seconde prothèse est déployée par rétraction du cathéter d'introducteur externe proximalement par rapport à l'introducteur principal de stent de façon à exposer la seconde prothèse depuis une extrémité distale du cathéter d'introducteur externe ;
**caractérisé en ce que** le cathéter d'introducteur externe comprend un orifice (66) à travers une paroi latérale du cathéter d'introducteur externe, l'orifice latéral étant proximal à la seconde prothèse et conçu pour laisser passer l'introducteur principal de stent à travers celui-ci.

2. Appareil introducteur de stent selon la revendication 1, dans lequel la première prothèse est montée amovible autour de l'élément poussoir.

3. Appareil introducteur de stent selon la revendication 2, dans lequel l'élément poussoir comprend une région de support de stent et un poussoir de stent, le poussoir de stent étant disposé proximalement à la région de support de stent et conçu pour venir en butée contre une extrémité proximale de la première prothèse, dans lequel en outre la première prothèse est montée amovible autour de la région de support de stent lorsqu'elle est disposée dans le cathéter d'introducteur interne.

4. Appareil introducteur de stent selon la revendication 1, dans lequel la seconde prothèse est déployée par rétraction du cathéter d'introducteur externe proximalement par rapport à l'élément poussoir de façon à mettre en prise la seconde prothèse avec l'élément poussoir et à exposer la seconde prothèse depuis une extrémité distale du cathéter d'introducteur externe.

5. Appareil introducteur de stent selon la revendication 4, dans lequel l'élément poussoir comprend un capuchon d'extrémité distale comprenant une bride et un élément de support, la bride étant conçue pour venir en butée contre une extrémité proximale de la seconde prothèse, l'élément de support étant conçu pour s'étendre vers un volume intérieur de la seconde prothèse.

6. Appareil introducteur de stent selon la revendication 1, dans lequel la seconde prothèse est déployée par rétraction du cathéter d'introducteur interne proximalement par rapport au cathéter d'introducteur interne de façon à mettre en prise la seconde prothèse avec le cathéter d'introducteur externe et à exposer la seconde prothèse depuis une extrémité distale du cathéter d'introducteur externe.

7. Appareil introducteur de stent selon la revendication 1, dans lequel l'élément poussoir comprend une lumière s'étendant à travers au moins une partie de celui-ci.

8. Appareil introducteur de stent selon la revendication 7, comprenant en outre un guide-fil, le guide-fil s'étendant à travers au moins une partie de la lumière.

9. Appareil introducteur de stent selon la revendication 1, dans lequel le cathéter d'introducteur externe comprend une extrémité distale, l'extrémité distale possédant un profil arrondi ou conique.

10. Appareil introducteur de stent selon la revendication 9, dans lequel l'extrémité distale est déployable pour permettre le passage de l'introducteur principal de stent à travers celle-ci.

11. Appareil introducteur de stent selon la revendication 10, dans lequel l'extrémité distale comprend une ou plusieurs fentes s'étendant au moins partiellement à travers une surface de celle-ci, la ou les fentes étant conçues pour permettre que l'extrémité distale se déploie afin de permettre le passage de l'introducteur principal de stent à travers celle-ci.
